# EUROPÄISCHE PATENTANMELDUNG

(11) **EP 3 747 399 A1**
(43) Veröffentlichungstag der Anmeldung: **09.12.2020**
(21) Anmeldenummer: 20178026.9
(22) Anmeldetag: 03.06.2020
(51) Int. Cl.: A61F 2/07, A61F 2/958

(54) **STENTGRAFT MIT DICHTUNGSELEMENT**

(30) Priorität: 04.06.2019 DE 102019115021
(71) Anmelder: Bentley InnoMed GmbH, 72379 Hechingen (DE)
(72) Erfinder: REIJNEN, Michel M.P.J., 6824HL Arnhem (NL)
(74) Vertreter: Hoefer & Partner Patentanwälte mbB

(57) **Zusammenfassung**

Die Erfindung betrifft einen Chimney-Stentgraft mit einem rohrförmigen Stentgraft-Körper (2) und mindestens einem Dichtungselement (3), wobei das Dichtungselement (3) den Stentgraft-Körper (2) in einem mittleren Abschnitt umschließt und Dichtungselement (3) eine schwamm- und/oder gewirrartige, elastische Struktur umfasst. Das Dichtungselement (3) sorgt für eine Abdichtung der zwischen dem Chimney-Stentgraft (1) und der Gefäßwand bzw. weiteren parallelen Stentgrafts entstehenden Lücken und verhindert so eine Leckage.

## Beschreibung

Die Erfindung betrifft einen Stentgraft mit einem Dichtungselement zum intravaskulären Einsatz insbesondere bei der Chimney-Technik.

Die Behandlung von abdominalen Aortenaneurysmen ist herausfordernd. Dies gilt insbesondere dann, wenn die Behandlung einen Gefäßabschnitt betrifft, der auch Viszeral- oder Nierenarterien einschließt. In Krankenhäusern, die auf entsprechende Eingriffe spezialisiert sind, sind die Mortalitäts- und Komplikationsraten zwar auch bei offenen Eingriffen vergleichsweise gering, diese positiven Ergebnisse sind jedoch nicht auf die Vielzahl von Zentren übertragbar.

Alternative minimalinvasive Interventionen, mit denen ein offener Eingriff und das damit verbundene Risiko von Wundinfektionen oder auch das Risiko einer Vollnarkose vermieden werden kann, sind im Stand der Technik bekannt.

Eine mögliche Form der minimalinvasiven Behandlung eines (Aorten-) Aneurysmas sieht die Überbrückung des geschwächten Gefäßabschnitts vor, innerhalb dessen sich das Aneurysma in Form einer Aussackung der geschwächten Gefäßwand ausbildet. Hierbei wird in der Regel ein Stentgraft (eine Gefäßprothese mit einem Stent und einem weitestgehend flüssigkeitsundurchlässigen Überzug) so platziert, dass dieser den geschwächten Gefäßabschnitt überbrückt und so einer möglichen Ruptur des Aneurysmas vorbeugt.

Nachteilig an dieser Technik ist, dass durch den Stentgraft häufig nicht nur der geschwächte Gefäßabschnitt, also das Aneurysma selbst, sondern auch weitere von dem betroffenen Gefäß abzweigende Seitenäste überdeckt und somit von der Blutzufuhr abgetrennt werden können. Dies kann zu einer Unterversorgung der von diesen Gefäßen abhängigen Organe und Gewebe führen.

Um diesen bekannten Komplikationen vorzubeugen sind derzeit insbesondere zwei Implantationstechniken etabliert. Zum einen kann der zu implantierende Stentgraft speziell an die Gefäßanatomie des Patienten angepasst sein. Solche individualisierten Stentgrafts beruhen entweder auf dem Baukastenprinzip oder werden vollständig neu angefertigt. Nachteilig an solchen individualisierten Systemen ist, dass sie recht teuer in der Anschaffung sind, vor allem wenn es sich um komplette Individualanfertigungen handelt. Zudem sind solche Systeme bei Notfällen nicht unmittelbar verfügbar, sondern müssen selbst im Fall von Baukastensystemen zunächst angepasst bzw. gefertigt werden. Bei vollständigen Individualanfertigungen kann dies Tage bis Wochen dauern.

Eine andere bekannte Implantationstechnik sieht vor, überdeckte Seitenäste durch jeweils eigene Stentgrafts wieder an die Blutversorgung des Hauptastes anzuschließen. Eine dieser Techniken, die sogenannte "Chimney-Technik", hat sich in letzter Zeit besonders bewährt.

Bei der Chimney-Technik wird der Anschluss der Seitenäste, die durch den im Hauptast platzierten Stentgraft überdeckten wurden, mit dem Blutstrom dadurch wieder hergestellt, dass weitere, dem Durchmesser des jeweiligen Seitenastes angepasste Stentgrafts parallel zu dem Hauptast-Stentgraft eingesetzt werden. Dabei reicht der in der Regel deutlich kleinerer Seitenast-Stentgraft vom Anfang des Hauptast-Stentgrafts, den er um wenige Millimeter überragt, bis in den Seitenast hinein. Das für diese Technik charakteristische "Überragen" des Hauptast-Stentgrafts durch den Seitenast-Stentgraft erinnert an einen Schornstein (englisch: *chimney*).

Die Chimney-Technik bietet viele Vorteile gegenüber anderen Interventionstechniken. So können beispielsweise für die gesamte Intervention gängige Stentgrafts verwendet werden und es müssen keine an die spezielle Gefäßanatomie angepassten Modelle gefertigt werden. Dies macht diese Technik nicht nur jederzeit - auch in Notfällen - verfügbar sondern zudem vergleichsweise kostengünstig.

Nachteilig an der Chimney-Technik mit herkömmlichen Stentgrafts ist jedoch vor allem, dass es zwischen den Stentgrafts und der Gefäßwand aufgrund der aneinander liegenden runden Formen der verschiedenen Stentgrafts zwangsläufig zu Zwischenräumen, Ritzen oder Spalten und in der Folge zu Leckagen kommen kann, wenn Blut durch diese Zwischenräume gelangt. Diese als Typ 1a Endoleckagen bezeichneten Lecks, können die Langlebigkeit eines solchen Arrangements von Stentgrafts negativ beeinflussen.

Ein weiterer Nachteil der Chimney-Technik mit herkömmlichen Stentgrafts besteht in den konkurrierenden radialen Kräften und den Expansionszwängen der parallel verlaufenden Stentgrafts. So besteht eine potentielle Gefahr, dass die Durchmesser der parallel verlaufenden Stentgrafts sich gegenseitig einengen, was wiederum zu einer gefährlichen Unterversorgung der jeweils angeschlossenen Organe und Gewebe führen kann. Diese Gefahr besteht besonders im Bereich der Abzweigung in den Seitenast.

Gerade bei dem Einsatz herkömmlicher Stentgrafts ist diese Gefahr besonders hoch, da deren Durchmesser tendenziell eher größer gewählt werden müssen, um die Gefahr von Leckagen zu minimieren. Genau dies führt jedoch gleichzeitig zu einem Aufeinandertreffen größerer Radialkräfte und somit zu einem höheren Risiko der beschriebenen Durchmesserreduktionen.

Entsprechend stehen sich bei der Auswahl eines geeigneten Stentgrafts für die Chimney-Technik grundsätzlich zwei Interessen entgegen: Einerseits soll der Stentgraft möglichst flexibel sein, um eine gute Passgenauigkeit und somit Abdichtung zu erzielen. Andererseits muss die Radialkraft aber auch groß genug sein, um dem Druck des eigentlichen Stentgrafts im Hauptast zu widerstehen. Eine größere Radialkraft führt jedoch immer auch zu einer geringeren Flexibilität und umgekehrt.

Um diese widerstreitenden Interessen des Anwenders und die sich entgegenstehenden Anforderungen an den Stentgraft zu überwinden, wurden im Stand der Technik verschiedene Vorrichtungen vorgeschlagen.

Zur Vermeidung bzw. Minimierung von Leckagen bei der Platzierung von Stentgrafts beispielsweise im Rahmen der Chimney-Technik beschreiben die WO 2017/114302 A1 und WO 2017/114305 A1 eine Vorrichtung, beim der zwei Stentgrafts derart kombiniert werden, dass ein erster Stentgraft zumindest teilweise von einem zweiten Stentgraft kragenartig umschlossen wird. Der zweite Stentgraft ist zumindest mit einem seiner Enden an der Außenseite des ersten Stentgrafts festgelegt. Eine vergleichbare Lösung wurde schon früher von der WO 2004/000167 A1 vorgeschlagen.

Nachteilig an diesen bekannten Stentgrafts bei ihrem Einsatz in der Chimney-Technik ist unter anderem, dass eine Stent-in-Stent-artige Situation geschaffen wird, die zu einer Versteifung des Gefäßes an dieser Stelle führt. Dies sollte jedoch unbedingt vermieden werden. Auch schaffen die bekannten Vorrichtungen keine ausreichende Abdichtung bis hin in die engsten Bereiche zwischen den aneinander liegenden Stents, sodass es trotz Einsatz dieser speziellen Stentgrafts immer noch zu Leckagen kommen kann.

Zudem kann es durch die Anordnung zweier sich kragenförmig überlappender Stents zu unerwünschten und nachteiligen Strömungsverläufen im Gefäß kommen, die auf Dauer die Offenheitsrate des Gefäßes oder des Stentgrafts negativ beeinflussen, also zu Gefäßverengungen oder in der Folge sogar zu Verstopfungen durch ungewollte Thrombenbildung führen.

Nicht zuletzt führt ein Stentgraft der bekannten Bauart durch die Stent-in-Stent Anordnung immer auch zu einem größeren Profil, also zu einem größeren Durchmesser, was den Einsatz in kleineren Gefäßen erschweren oder sogar unmöglich machen kann.

Es ist daher Aufgabe der Erfindung einen Stentgraft, nachfolgend der Unterscheidbarkeit halber als "Chimney-Stentgraft" bezeichnet, zur Verfügung zu stellen, der die genannten Nachteile überwindet und insbesondere zur Verwendung bei der Chimney-Technik geeignet ist.

Gelöst wird diese Aufgabe durch eine Erfindung mit den Merkmalen des Anspruchs 1. Vorteilhafte Ausgestaltungen sind jeweils Gegenstand der abhängigen Ansprüche. Es ist darauf hinzuweisen, dass die in den Ansprüchen einzeln aufgeführten Merkmale auch in beliebiger und technologisch sinnvoller Weise miteinander kombiniert werden können und somit weitere Ausgestaltungen der Erfindung aufzeigen.

Ein erfindungsgemäßer Chimney-Stentgraft umfasst einen Stentgraft-Körper und zumindest ein Dichtungselement.

Sofern von einem "Stentgraft-Körper" als Teil des erfindungsgemäßen Chimney-Stentgrafts gesprochen wird, so soll, um eine Unterscheidung zu bekannten Stentgrafts zu schaffen, damit der Stentgraft-Anteil des erfindungsgemäßen Chimney-Stentgrafts ohne Dichtungselemente gemeint sein.

Aus Gründen der besseren Verständlichkeit soll nachfolgend zudem immer nur von "einem" oder "dem" Dichtungselement gesprochen werden, auch wenn erfindungsgemäß mehr als ein Dichtungselement vorgesehen sein kann.

Der Stentgraft-Körper an sich entspricht in seinem Aufbau im Wesentlichen bekannten Stentgrafts (Gefäßprothesen) mit einem expandierbaren Stent (dem Metallgerüst) und einer an dem Stent festgelegten Hülle (dem "Graft"). Die Hülle umfasst in der Regel eine im Wesentlichen flüssigkeitsundurchlässige Polymerhülle, die den Stent zumindest teilweise umgibt.

Der Stentgraft-Körper kann ballonexpandierbar oder selbstexpandierbar sein, wobei ballonexpandierbare Stentgrafts wegen ihrer höheren Radialkraft zu bevorzugen sind. Die Hülle kann die für Stentgrafts typischen Materialien wie verschiedene Polymere und insbesondere PTFE umfassen. Die Hülle kann auch mehrschichtig aufgebaut sein und zusätzliche Beschichtungen umfassen, wie beispielsweise antithrombogene, antirestenotische oder antibiotische Eigenschaften aufweisen, um nur einige zu nennen. Hier sind dem Fachmann eine Vielzahl an Materialien und Wirkstoffen bekannt, aus denen er die für den jeweiligen Zweck geeigneten Materialien und Wirkstoffe auswählen kann.

Das Dichtungselement des erfindungsgemäßen Chimney-Stentgrafts ist im mittleren Abschnitt des Stentgraft-Körpers vorgesehen. Der mittlere Abschnitt definiert sich erfindungsgemäß als der Abschnitt des Stentgraft-Körpers, der zum distalen und proximalen Ende des Stentgraft-Körpers jeweils einen Abstand von 1 bis 15 mm und vorzugsweise von 5 bis 10 mm einhält. Sofern mehrere Dichtungselemente vorgesehen sind, so sind vorzugsweise insbesondere zwei Dichtungselemente im mittleren Abschnitt des Stent-Körpers vorgesehen.

Das Dichtungselement ist vorzugsweise im Wesentlichen hohlzylinder- bzw. ringförmig ausgebildet. Den jeweiligen Anforderungen entsprechend können jedoch auch Dichtungselemente sonstiger Formen vorgesehen sein, beispielsweise sich kegelartig verjüngende, hantelförmig ausgestellte, gewellte, runde oder gänzlich amorphe Dichtungselemente. Solche Formen können in verschieden ausgebildeten Gefäßanatomien jeweils vorteilhaft sein. Eine amorphe Form kann beispielsweise beim Einsatz des Chimney-Stentgrafts in unregelmäßig geformten Gefäßen vorteilhaft sein. Hier wird der Fachmann ohne erfinderisch tätig zu werden die Form wählen, die eine für den Einsatz bestmögliche Abdichtung verspricht.

Die Enden der Wandung des Dichtungselements sind vorzugsweise nahezu eckig ausgestaltet, wobei auch Ausführungsformen mit abgerundeten oder auch nur teilweise abgerundeten Enden der Wandung denkbar sind. Auch hier entscheidet der Fachmann sich für die Form, die für den jeweiligen Einsatzzweck die bestmögliche Abdichtung verspricht.

Der innere Durchmesser des frei expandierten Dichtungselements entspricht im Wesentlichen dem äußeren Durchmesser des frei expandierten Stentgraft-Körpers. Das Dichtungselement lässt sich wie auch der Stentgraft-Körper auf einen kleineren Durchmesser komprimieren. Der innere Durchmesser des komprimierten Dichtungselements entspricht dann im Wesentlichen dem äußeren Durchmesser des komprimierten Stentgraft-Körpers.

Sofern der Begriff "frei expandiert" verwendet wird, so ist hierunter erfindungsgemäß ein Zustand zu verstehen, bei dem das jeweilige Element, also der Chimney-Stentgraft in seiner Gesamtheit, der Stentgraft-Körper allein oder das Dichtungselement allein, den jeweils maximal vorgesehenen expandierten Zustand ohne äußere Zwänge bei der für den Einsatz vorgesehenen Temperatur erreicht hat. Dies ist bei einem Ballon expandierbaren Element beispielsweise die Aufweitung des Elements mittels Ballonexpansion auf den vorgesehenen nominalen Durchmesser und bei einem selbstexpandierenden Element die Aufweitung außerhalb eines Zuführelements ohne äußere Zwänge jeweils bei einer Temperatur, die der durchschnittlichen menschlichen Körpertemperatur im Gefäß entspricht. Entsprechend beziehen sich etwaige Dimensionsangaben aus Gründen der Nachvollziehbarkeit und Objektivität auf den frei expandierten Zustand soweit nicht anders erwähnt.

Der maximale äußere Durchmesser des frei expandierten Dichtungselements liegt zwischen 1 und 10 mm, vorzugsweise zwischen 2 und 8 mm und insbesondere zwischen 3 und 6 mm größer als der innere Durchmesser des frei expandierten Dichtungselements.

Das Dichtungselement ist vorzugsweise konzentrisch um den Stentgraft-Körper vorgesehen, es sind jedoch auch Ausführungsformen denkbar, bei denen das Dichtungselement exzentrisch um den Stentgraft-Körper angeordnet ist. Eine solche exzentrische Anordnung des Dichtungselements kann beispielsweise beim Einsatz des Chimney-Stentgraft in unregelmäßig geformten Gefäßen vorteilhaft sein. Eine konzentrische Anordnung des Dichtungselements hingegen sollte den größten Bereich von Standardinterventionen abdecken können, bei denen man einer gewöhnlichen Gefäßmorphologie begegnet.

Wichtig ist bei der Anbringung des Dichtungselements, egal ob diese exzentrisch oder konzentrisch erfolgt, dass eine ausreichende Anpassung an die vorgegebene Umgebung möglich ist.

Das Dichtungselement ist mit dem Stentgraft-Körper verbunden und passt sich dem Umfang des Stentgraft-Körpers im komprimierten Zustand ebenso wie im expandierten Zustand an. Das Dichtungselement kann temporär und/oder dauerhaft mit dem Stentgraft-Körper verbunden sein.

Unter einer temporären Verbindung von Stentgraft-Körper und Dichtungselement ist erfindungsgemäß eine solche Verbindung zu verstehen, die während bzw. nach der Platzierung und Expansion des Chimney-Stentgrafts lösbar vorgesehen ist. Entsprechend bleibt nach der Implantation die relative Anordnung von Stentgraft-Körper und Dichtungselement zueinander auf Dauer vor allem durch die aufeinander wirkenden Drücke zwischen Stentgraft-Körper und Dichtungselement einerseits sowie (Hauptast-) Stentgraft und Gefäßwand andererseits erhalten.

Beispiele für eine solche temporäre Verbindung sind beispielsweise das Vernähen mit bioresorbierbaren Fäden oder das Verkleben mit bioresorbierbaren Klebstoffen von Dichtungselement und zumindest Teilen des Stentgraft-Körpers.

Auch sind temporäre Verbindungen denkbar, bei denen das Dichtungselement mithilfe von insbesondere ringförmigen Halteelementen an dem Stentgraft-Körper angeordnet ist, wobei die ringförmigen Haltelemente vorzugsweise Sollbruchstellen aufweisen, die bei Ausdehnung des Chimney-Stentgrafts reißen und so eine Aufweitung des Chimney-Stentgrafts ermöglichen. Anstelle von ringförmigen Halteelementen könnten hier beispielsweise auch eine Schlauchfolie oder Fäden vorgesehen sein, die bei der Expansion entlang einer perforierten Naht aufreißen. Dem Fachmann sind weitere Techniken bekannt, aus denen er eine geeignete auswählt.

Unter einer dauerhaften Verbindung von Stentgraft-Körper und Dichtungselement ist erfindungsgemäß hingegen eine solche Verbindung zu verstehen, die auch nach der Platzierung und Expansion des Chimney-Stentgrafts weiter besteht. Dabei wird die relative Anordnung von Stentgraft-Körper und Dichtungselement zueinander auch oder vor allem durch die dauerhafte Verbindung von Stentgraft-Körper und Dichtungselement aufrechterhalten.

Beispiele für eine solche dauerhafte Verbindung sind beispielsweise das Vernähen, Verkleben oder Verschweißen, auch Ultraschall-Schweißen, von Dichtungselement und zumindest Teilen des Stentgraft-Körpers. Der Fachmann wählt hier eine geeignete Technik aus.

Kombinationen von dauerhaften und temporären Verbindungen sind denkbar. So kann das Dichtungselement beispielsweise dauerhaft mit dem Stentgraft-Körper verbunden sein und zusätzliche ringförmigen Halteelemente mit Sollbruchstellen umfassen, die um das Dichtungselement herumreichen und so dessen komprimierten Zustand fixieren. Der Fachmann wird anhand dieser Beispiele leicht weitere geeignete Ausführungsformen auswählen können, ohne dabei erfinderisch tätig werden zu müssen.

Um die Platzierung des Chimney-Stentgrafts zu erleichtern, können neben den bekannten röntgendichten Markern an den Enden des Stentgraft-Körpers zusätzliche röntgendichte Marker im Bereich des Dichtungselements vorgesehen sein. Diese zusätzlichen Marker können beispielsweise an entsprechenden Stellen des Stentgraft-Körpers, am Dichtungselement oder gegebenenfalls an vorhandenen Halteelementen vorgesehen sein. Die zusätzlichen Marker vereinfachen die Platzierung des erfindungsgemäßen Chimney-Stentgrafts, indem sie dem Behandler die Bereiche anzeigen, die für die gewünschte Abdichtung relevant sind.

Materialien für entsprechende röntgendichte Marker sind dem Fachmann bekannt.

Das Dichtungselement dient dazu, Durchlässe zwischen dem Stentgraft-Körper einerseits und der Gefäßwand sowie den übrigen Stentgrafts wie beispielsweise dem (Hauptast-) Stentgraft andererseits abzudichten. Hierzu umfasst das Dichtungselement ein biokompatibles Material, das sich flexibel an die äußere Umgebung anpassen bzw. in die äußere Umgebung einfügen kann.

Es ist insbesondere wichtig, dass das Material für das Dichtungselement einerseits stark kompressibel ist, sich andererseits aber auch selbst nach längerer Zeit wieder auf den ursprünglichen nicht komprimierten Zustand aufweitet. Der Zustand der Kompression sollte demnach möglichst vollständig reversibel sein.

Wesentlich für die Erfindung ist dabei, dass das Dichtungselement eine schwamm- und/oder gewirrartige und dabei gleichzeitig elastische Struktur aufweist. Unter einer solchen Struktur sind erfindungsgemäß Strukturen zu verstehen, die ein eigenes Volumen und eine Kompaktheit aufweisen und so einen Durchfluss von Flüssigkeiten weitestgehend unterbinden können, jedoch gleichzeitig ausreichend kompressibel sind, um in einem Katheter platziert zu werden. Eine schwamm- und/oder gewirrartige Struktur im Sinne dieses Patents kann somit sowohl eine poröse als auch eine stark verzweigte bzw. verästelte Struktur sein. Ein geeignetes Gewirr kann beispielsweise aus nur einem oder wenigen Filamenten geformt sein, es sind jedoch auch Gewirre aus einer Vielzahl von Filamenten bis hin zu vliesartigen Strukturen denkbar.

Geeignete Materialien für das Dichtungselement umfassen unter anderem insbesondere Formgedächtnismaterialien, wie beispielsweise Formgedächtnispolymere (FGP) oder Formgedächtnislegierungen (FGL), oder Federstahle. Bekannte Materialien sind hier beispielsweise Nitionol und andere.

Weitere geeignete Materialien für das Dichtungselement umfassen auch sonstige flexible Materialien, insbesondere elastische Polymere (Elastomere), die nach einer erfolgten Kompression wieder einen expandierten Zustand einnehmen können. Hierzu zählen verschiedenste Polymere wie beispielsweise verschiedene Polyurethan-Verbindungen.

Entsprechend kann eine erfindungsgemäße schwammartige Struktur des Dichtungselements durch verschiedenste Materialien und Materialkombinationen erreicht werden. Mögliche Strukturen umfassen beispielsweise geflochtene und nicht geflochtene Materialien, Vliese oder auch mehrschichtig angeordnete Materialien oder Materialkombinationen oder Kombinationen hieraus.

Vorzugsweise weist das Dichtungselement neben der bestimmten Ausformung oder dem Einsatz bestimmter Strukturen, die eine Thrombenbildung fördern, noch zusätzliche thrombogene Eigenschaften auf. Das Dichtungselement kann entsprechend aus thrombogenen Materialien gefertigt sein, wie beispielsweise verschiedenen Polyamide wie PET oder Nylon, um nur einige zu nennen. Auch kann das Dichtungselement mit einem thrombogenen Mittel beschichtetet sein. Hier steht dem Fachmann eine Vielzahl bekannter thrombogener Beschichtungsmittel zu Verfügung. Vorteilhaft an der Verwendung thrombenfördernder Strukturen sowie thrombogener Materialien oder Beschichtungen ist, dass eine zusätzliche Abdichtung des Dichtungselements durch einen sich an dem thrombogenen Material bildenden Thrombus erreicht wird. Das Dichtungselement wird entsprechend in seinem passgenauen Zustand und seiner Abdichtung durch die Thrombenbildung verstärkt.

Ein Dichtungselement mit einer solchen erfindungsgemäßen Struktur hat wesentliche Vorteile gegenüber den aus dem Stand der Technik bekannten schirmartigen Abdichtungselementen.

So ist durch das für die Funktion wesentliche Dichtungselement ein dichterer Abschluss möglich als mit den bekannten schirmartigen Systemen, denn es kann sich wesentlich dichter auch in die feinsten Spalten ausdehnen. Der erfindungsgemäße Chimney-Stentgraft hält durch das spezielle Dichtungselement auch dem gegen die Dichtung wirkenden Blutstrom besser stand. Eine schirmartige Konstruktion birgt immer das Risiko, bei zu großen Drücken umzuklappen und somit einen Durchfluss zu ermöglichen. Erst durch den durch die Kompaktheit des erfindungsgemäßen Dichtungselements besonders dichten und stabilen Abschluss wird eine Leckage auch dauerhaft zuverlässig unterbunden.

Der erfindungsgemäße Chimney-Stentgraft kann auch mit einer deutlich größeren Radialkraft des Stentgraftkörpers konstruiert und hergestellt werden, da die Abdichtung, die eigentlich eine erhöhte Flexibilität des Stentgrafts erfordert, nun durch das flexible separate Dichtungselement erzielt wird.

Diese Eigenschaften machen den Einsatz des erfindungsgemäßen Chimney-Stentgrafts beispielsweise auch im Zusammenhang mit endovaskulären Aortenreparaturen (EVAR) interessant, da hier genau diese Eigenschaften einer guten Abdichtung verbunden mit einer hohen Radialkraft gefordert sind.

Zudem wird durch das erfindungsgemäße Dichtungselement gegenüber den bekannten Vorrichtungen auch eine Stent-in-Stent ähnliche Situation vermieden, die zu einer Versteifung des Gefäßes an dieser Stelle führt, welche unbedingt vermieden werden sollte.

Nicht zuletzt kann der erfindungsgemäße Chimney-Stentgraft mit einem kleineren Profil hergestellt und somit auch in kleineren Gefäßen eingesetzt werden.

Auch wenn die vorliegende Erfindung durchweg als "Chimney-Stentgraft" bezeichnet wird, so ist diese Bezeichnung lediglich der Einfachheit halber gewählt und nur beispielhaft für einen möglichen Einsatz der erfindungsgemäßen Vorrichtung. Die Bezeichnung ist somit keinesfalls einschränkend für eine entsprechende Verwendung bei der Chimney-Technik zu verstehen. Der Fachmann wird problemlos weitere Einsatzmöglichkeiten eines erfindungsgemäßen Stentgraft-Körpers mit Dichtungselement im klinischen Alltag finden, für die dieser ebenso gut geeignet ist wie im Rahmen der Chimney-Technik.

Die Erfindung sowie das technische Umfeld werden nachfolgend anhand der Figuren näher erläutert. Es ist darauf hinzuweisen, dass die Figuren eine besonders bevorzugte Ausführungsvariante der Erfindung zeigen. Die Erfindung ist jedoch nicht auf die gezeigte Ausführungsvariante beschränkt. Insbesondere umfasst die Erfindung, soweit es technisch sinnvoll ist, beliebige Kombinationen der technischen Merkmale, die in den Ansprüchen aufgeführt oder in der Beschreibung als erfindungsrelevant beschrieben sind.

Es zeigen:
- Fig. 1: Eine erste Ausführungsform des erfindungsgemäßen Chimney-Stentgrafts
- Fig. 2: Detailansichten des Dichtungselements
- Fig. 3: Querschnitt durch ein Blutgefäß mit einem Hauptast-Stentgraft und zwei erfindungsgemäßen Chimney-Stentgrafts

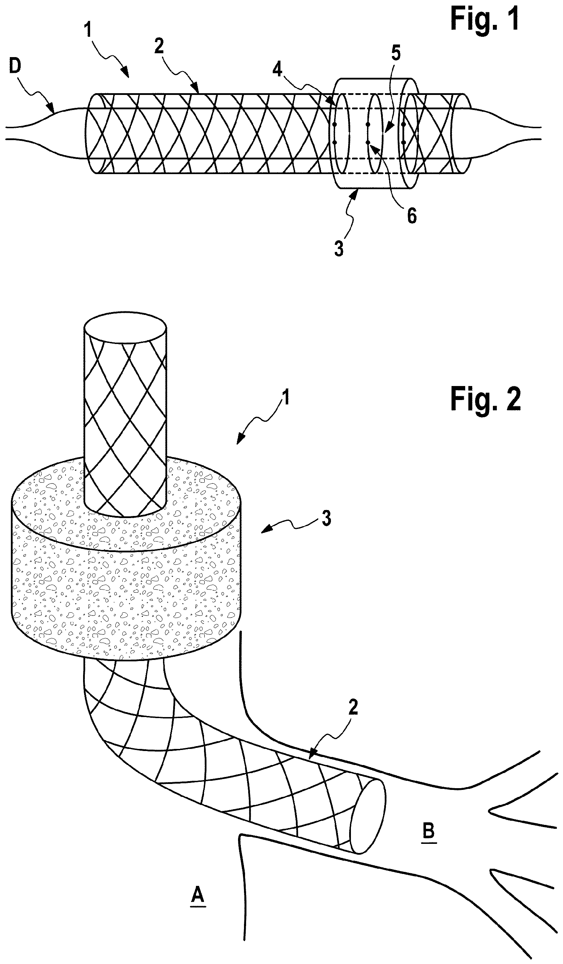

Fig. 1 zeigt eine erste Ausführungsform eines erfindungsgemäßen Chimney-Stentgrafts 1. Der Chimney-Stentgrafts 1 ist auf einen Ballonkatheter D gecrimpt und umfasst einen Stentgraft-Körper 2 und ein Dichtungselement 3.

Das Dichtungselement 3 umfasst ein elastisches, schwammartiges Material, das wie angedeutet eine poröse oder stark verzweigte bzw. verästelte oder faserige Struktur aufweist. In jedem Fall ist das Dichtungselement 3 massiv und voluminös in dem Sinne, dass es vor allem nicht aus nur einer dünnen gegebenenfalls durch Aufspannelemente gehaltenen Membran besteht.

Das Dichtungselement 3 ist durch ringförmige Haltelemente 4 mit dem Stentgraft-Körper 2 verbunden. Die Haltelemente 4 umfassen Sollbruchstellen 5, die bei Expansion des Stentgraft-Körpers 2 brechen. Im Bereich des Dichtungselements 3 können zusätzliche röntgendichte Marker 6 vorgesehen sein, die eine Platzierung des Chimney-Stentgrafts 1 erleichtern, indem sie die Position des Dichtungselements 3 anzeigen. Diese Marker 6 können beispielsweise an dem Stentgraft-Körper 2, dem Dichtungselement 3 oder an zumindest einem der Halteelemente 5 vorgesehen sein.

Fig. 2 zeigt beispielhaft einen Längsschnitt durch ein Blutgefäß A (den Hauptast), in dem ein erfindungsgemäßer Chimney-Stentgrafts 1 im Hauptast A platziert wurde. Es ist erkennbar, dass der Chimney-Stentgraft 1 distal in den Seitenast B hineinragt und diesen so an die Blutzufuhr über den Hauptast A anbindet.

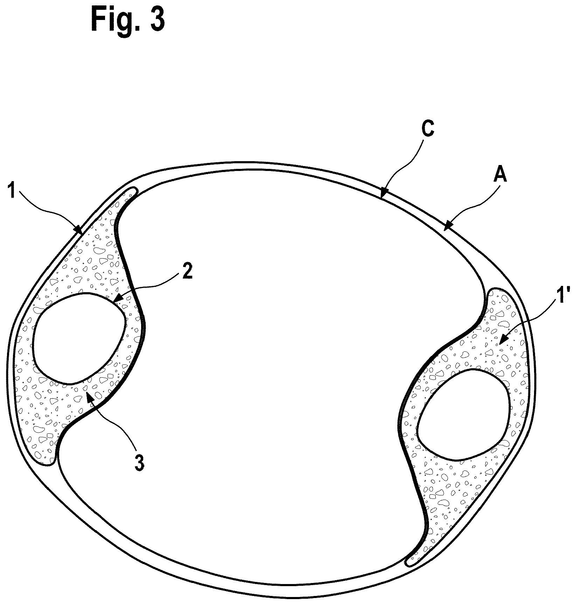

Fig. 3 zeigt einen Querschnitt durch ein Blutgefäß A (den Hauptast) an einer Stelle, an der ein Hauptast-Stentgraft C und zwei erfindungsgemäße Chimney-Stentgrafts 1, 1' parallel zueinander platziert wurden. Der Stentgraft-Körper 2 ist an dieser Stelle von dem Dichtungselement 3 umgeben. Das Dichtungselement 3 füllt die zwischen Gefäßwand, Stentgraft-Körper 2 und Hauptast-Stentgraft C entstandene Lücken vollständig aus und verhindert so eine Leckage.

### Bezugszeichenliste

- 1,1': Chimney-Stentgraft
- 2: Stentgraft-Körper
- 3: Dichtungselement
- 4: Halteelement
- 5: Sollbruchstelle
- 6: Marker
- A: Hauptast
- B: Seitenast
- C: Hauptast-Stentgraft
- D: Ballonkatheter

## Patentansprüche

1. Chimney-Stentgraft umfassend einen rohrförmigen Stentgraft-Körper (2) und mindestens ein Dichtungselement (3), wobei das Dichtungselement (3) den Stentgraft-Körper (2) in seinem mittleren Abschnitt umschließt, **dadurch gekennzeichnet, dass** das Dichtungselement (3) eine schwamm- und/oder gewirrartige, elastische Struktur umfasst.

2. Vorrichtung nach Anspruch 1, **dadurch gekennzeichnet, dass** das Dichtungselement (3) hohlzylinder- oder ringförmig ausgebildet ist.

3. Vorrichtung nach Anspruch 1 oder 2, **dadurch gekennzeichnet, dass** die schwamm- und/oder gewirrartige Struktur des Dichtungselements (3) mindestens ein Material umfasst ausgewählt aus der Gruppe enthaltend Formgedächtnispolymere (FGP), Formgedächtnislegierungen (FGL), Federstahle und Elastomere.

4. Vorrichtung nach einem der vorherigen Ansprüche, **dadurch gekennzeichnet, dass** das Dichtungselement (3) mindestens ein Material umfasst, das eine thrombogene Beschichtung umfasst und/oder das thrombogene Eigenschaften aufweist.

5. Vorrichtung nach einem der vorherigen Ansprüche, **dadurch gekennzeichnet, dass** das Material oder die Materialien und/oder die Strukturen mehrschichtig angeordnet sind.

6. Vorrichtung nach einem der vorherigen Ansprüche, **dadurch gekennzeichnet, dass** der äußere Durchmesser des frei expandierten Dichtungselements (3) um 1 bis 10, vorzugsweise 2 bis 6 und insbesondere 3 bis 5 mm größer ist als der äußere Durchmesser des frei expandierten Stentgraft-Körpers (2).

7. Vorrichtung nach einem der vorherigen Ansprüche, **dadurch gekennzeichnet, dass** der innere Durchmesser des Dichtungselements (3) dem äußeren Durchmesser des Stentgraft-Körpers (2) entspricht.

8. Vorrichtung nach einem der vorherigen Ansprüche, **dadurch gekennzeichnet, dass** der Stentgraft-Körper (2) einen selbstexpandierenden oder ballonexpandierbaren Stent und eine an dem Stent angeordnete Hülle umfasst.

9. Vorrichtung nach einem der vorherigen Ansprüche, **dadurch gekennzeichnet, dass** das Dichtungselement (3) an der Hülle oder dem Stent des Stentgraft-Körpers (2) festgelegt ist.

10. Vorrichtung nach einem der vorherigen Ansprüche, **dadurch gekennzeichnet, dass** im Bereich des Dichtungselements (3) röntgendichte Marker vorgesehen sind.
